(19) Europäisches Patentamt
European Patent Office
Office européen des brevets

(11) **EP 4 120 909 B1**

(12) **EUROPEAN PATENT SPECIFICATION**

(45) Date of publication and mention of the grant of the patent:
**21.05.2025 Bulletin 2025/21**

(21) Application number: **21717709.6**

(22) Date of filing: **17.03.2021**

(51) International Patent Classification (IPC):
*A61B 5/107* (2006.01)  *A61B 5/00* (2006.01)

(52) Cooperative Patent Classification (CPC):
**A61B 5/1074; A61B 5/0053; A61B 5/4528; A61B 5/702**

(86) International application number:
**PCT/US2021/022791**

(87) International publication number:
**WO 2021/188693 (23.09.2021 Gazette 2021/38)**

(54) **FOOT STRUCTURE AND FUNCTION ASSESSMENT DEVICE AND METHODS OF USING SAME**

FUSSSTRUKTUR UND FUNKTIONSBEURTEILUNGSVORRICHTUNG UND VERFAHREN ZUR VERWENDUNG DAVON

STRUCTURE DE PIED ET DISPOSITIF D'ÉVALUATION DE FONCTION ET PROCÉDÉS D'UTILISATION ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2020 US 202062990816 P**

(43) Date of publication of application:
**25.01.2023 Bulletin 2023/04**

(73) Proprietor: **New York Society for the Relief of the Ruptured and Crippled, maintaining the Hospital for Special Surgery
New York, NY 10021 (US)**

(72) Inventors:
• **MORGAN, Oliver**
  **London E11 4PP (GB)**
• **HILLSTROM, Howard**
  **New York, NY 10044 (US)**
• **ELLIS, Scott, J.**
  **New York, NY 10128 (US)**
• **SONG, Jinsup**
  **Bala Cynwyd, PA 19004 (US)**
• **TURNER, Robert**
  **New York, NY 10128 (US)**
• **DELAND, Jonathan**
  **New York, NY 10021 (US)**
• **HILLSTROM, Rajshree**
  **New York, NY 10044 (US)**

(74) Representative: **Cameron Intellectual Property Ltd
Moncrieff House
69 West Nile Street
Glasgow G1 2QB (GB)**

(56) References cited:
**WO-A1-2017/171660**

• **GLASOE WARD MYLO ET AL: "The Reliability and Validity of a First Ray Measurement Device", FOOT AND ANKLE INTERNATIONAL, vol. 21, no. 3, 1 March 2000 (2000-03-01), US, pages 240 - 246, XP055813237, ISSN: 1071-1007, DOI: 10.1177/107110070002100310**

**Description**

FIELD OF THE INVENTION

The present invention relates generally to a medical device for assessing foot structure and function. In particular, the present invention relates to a medical device operable to assess first ray position, first ray mobility, arch height index, arch height flexibility, arch stiffness, and first metatarsophalangeal joint flexibility of a patient's foot.

BACKGROUND OF THE INVENTION

The "first ray" refers to a segment of a patient's foot composed of a navicular, medial cuneiform, first metatarsal, and hallux bones. Aberrant first ray structure and function have been implicated in onset and progression of osteoarthritis in the first metatarsophalangeal joint, sometimes referred to as hallux rigidus. Specifically, investigation is ongoing regarding the impact of hypermobility of the first ray in the superior direction, measured by first ray mobility, on hallux rigidus.

Conventional assessment devices are operable to quantify first ray mobility objectively. However, such devices tend to yield inaccurate measurements, in part due to requiring manual force during at least part of the examination, thereby potentially adding undesired variability to the measurement. Conventional devices can also be complicated and bulky, thereby limiting their utility in a clinical or research setting.

The publication GLASOE WARD MYLO ET AL: "The Reliability and Validity of a First Ray Measurement Device", FOOT AND ANKLE INTERNATIONAL, vol. 21, no. 3, 1 March 2000 (2000-03-01), pages 240-246,US ISSN: 1071-1007, DOI: 10.1177/107110070002100310 and the document WO 2017/171660 A1 disclose devices for measuring mobility of the first ray of a subject 's foot.

SUMMARY OF THE INVENTION

In accordance with an exemplary embodiment of the subject disclosure, a foot structure and function assessment device as defined in claim 1 is provided. The device includes a base having a top surface for supporting a patient's foot, a clamp attached to the base for immobilizing a second metatarsal of the patient's foot, a vertical measurement mechanism carried by the base for measuring displacement of a first ray of the patient's foot, an actuation member for vertically moving the first ray, and a controller operatively in communication with the actuation member and configured to displace the first ray by applying a first vertical load to the first ray using the actuation member while the second metatarsal is immobilized, where the first vertical load is associated with a first ray position of the patient's foot, and displace the first ray by applying a second vertical load to the first ray using the actuation member while the second metatarsal is immobilized, where the second vertical load is associated with a first ray mobility of the patient's foot.

The controller is further configured to measure the first ray position of the patient's foot under the applied first vertical load, and to measure the first ray mobility of the patient's foot under the applied second vertical load. The first vertical load can be about 3 N to about 10 N, and the second vertical load can be about 45 N to about 55 N. The actuation member further includes a first movable pointer adjacent the vertical measurement mechanism for determining first ray position, and a second movable pointer adjacent the vertical measurement mechanism for determining first ray mobility. The device can further include a heel cup movably attached to the base for contacting a patient's rearfoot, and a horizontal measurement mechanism carried by the base for measuring a foot length of the patient's foot. The device can further include an actuator rigidly coupled to the actuation member, where the actuator is operable to move the actuation member. The clamp can be laterally spaced from the actuation member. The clamp can be spaced from the actuation member such that the clamp spatially corresponds to and can be operable to immobilize a second metatarsal of the patient's foot as the actuation member displaces the first ray. The actuation member can contact a plantar surface of the first ray. The device can further include an arch height measurement mechanism laterally spaced from the actuation member and movable to about one-half of a total foot length of the patient's foot for measuring an arch height of the patient's foot. The device can further include a torque member laterally spaced from the actuation member and movable to a first metatarsal head of the patient's foot for applying a moment to the patient's foot about an axis of rotation of a first metatarsophalangeal joint. The vertical measurement mechanism can include a vertical graticule, a position sensor, or a linear variable differential transformer

In accordance with another exemplary embodiment, the subject disclosure provides a method for assessing foot structure and function as defined in claim 10.

The applied first vertical load can be about 5 N and the applied second vertical load can be about 50 N. The measuring the first ray position includes using a first movable pointer adjacent the vertical measurement mechanism to measure the first ray position, and the measuring the first ray mobility can include using a second movable pointer adjacent the vertical measurement mechanism to measure the first ray mobility. The method can further include measuring, using a foot length measurement mechanism or an actuator laterally spaced from the actuation member, a foot length of the patient's foot, measuring, using an arch height measurement mechanism laterally spaced from the actuation member, an arch height of the patient's foot, and determining an arch height index of the patient's foot based on normalizing the arch height using the foot length. The method can further include determining, using an arch height measurement mechanism laterally spaced from the actuation member, an arch height of the patient's foot while the patient is seated, determining, using the arch height measurement mechanism, an arch height of the patient's foot while the patient is standing, measuring, using a scale laterally spaced from the actuation member, a body weight of the patient, and determining an arch height flexibility of the patient's foot based on the seated arch height, the standing arch height, and the body weight. The method can further include determining, using an arch height measurement mechanism laterally spaced from the actuation member, an arch height index of the patient's foot while the patient is seated, determining, using the arch height measurement mechanism, an arch height index of the patient's foot while the patient is standing, measuring, using a scale laterally spaced from the actuation member, a body weight of the patient, and determining an arch stiffness of the patient's foot based on the body weight, the seated arch height, and the standing arch height. The method can further include applying, using a torque member laterally spaced from the actuation member, a joint moment to a first metatarsophalangeal joint of the patient's foot, and determining a first metatarsophalangeal joint flexibility of the patient's foot under the applied joint moment. The step of determining the first metatarsophalangeal joint flexibility can include determining the first metatarsophalangeal joint flexibility based on determining a slope of a first metatarsophalangeal joint flexibility curve. The step of determining the first metatarsophalangeal joint flexibility can include determining an early first metatarsophalangeal joint flexibility or a late first metatarsophalangeal joint flexibility.

BRIEF DESCRIPTION OF THE SEVERAL VIEWS OF THE DRAWINGS

The foregoing summary, as well as the following detailed description of the exemplary embodiments of the invention, will be better understood when read in conjunction with the appended drawings. For the purpose of illustrating the present disclosure, there are shown in the drawings exemplary embodiments. It should be understood, however, that the subject application is not limited to the precise arrangements and instrumentalities shown.

In the drawings:

FIG. 1 illustrates a front perspective view of an exemplary foot structure and function assessment device in accordance with an exemplary embodiment of the subject disclosure;

FIG. 2 illustrates a rear perspective view of the foot structure and function assessment device of FIG. 1;

FIG. 3 illustrates a top plan view of the foot structure and function assessment device of FIG. 1;

FIG. 4 illustrates a lateral perspective view of a clamp assembly of the foot structure and function assessment device of FIG. 1;

FIG. 5 illustrates a lateral perspective view of an actuation member and load cell assembly of the foot structure and function assessment device of FIG. 1 with certain components omitted and/or in phantom for purposes of illustration;

FIG. 6 illustrates a top plan view of an exemplary foot structure and function assessment device in accordance with another exemplary embodiment of the subject disclosure;

FIG. 7 illustrates a flowchart of an exemplary method for assessing first ray mobility and position using the foot structure and function assessment device of FIGS. 1 or 6;

FIG. 8 illustrates a flowchart of an exemplary method for assessing arch height index using the foot structure and function assessment device of FIG. 6;

FIG. 9 illustrates a flowchart of an exemplary method for assessing arch height flexibility using the foot structure and function assessment device of FIG. 6;

FIG. 10 illustrates a flowchart of an exemplary method for assessing arch stiffness using the foot structure and function assessment device of FIG. 6;

FIG. 11 illustrates a flowchart of an exemplary method for assessing first MTP joint flexibility using the foot structure and function assessment device of FIG. 6; and

FIG. 12 illustrates an exemplary curve for assessing first MTP joint flexibility using the foot structure and function assessment device of FIG. 6.

## DETAILED DESCRIPTION OF THE DISCLOSURE

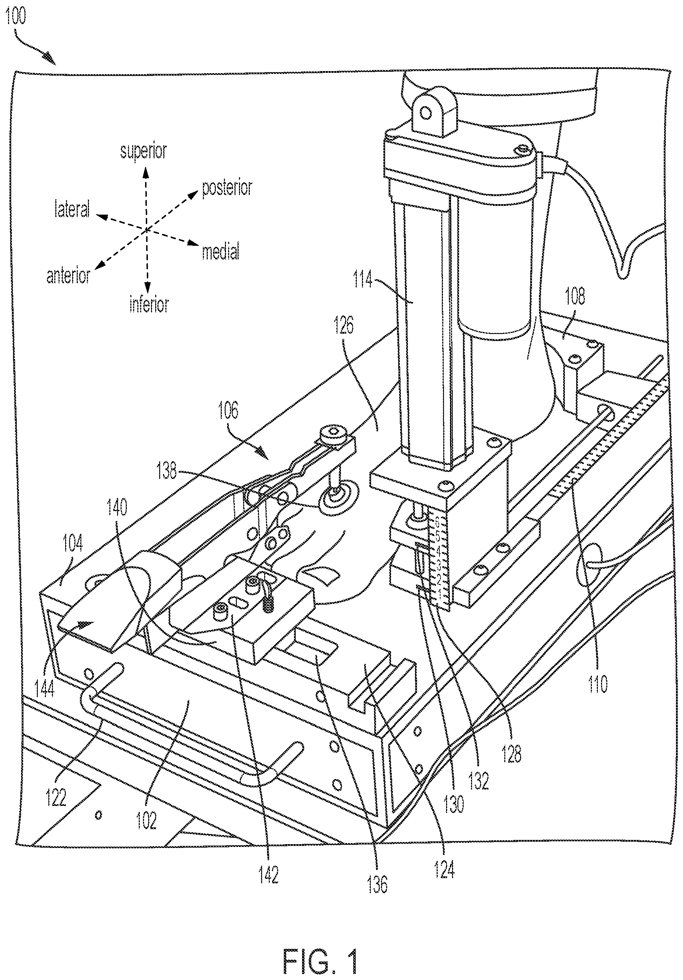

FIGS. 1-3 illustrate an exemplary embodiment of a foot structure and function assessment device 100 in accordance with the subject disclosure. The device 100 allows a user to assess a foot structure and function of a patient. The foot structure and function assessments include a first ray position, a first ray mobility, and optionally one or more of an arch height index, an arch height flexibility, an arch stiffness, and a first metatarsophalangeal (MTP) joint flexibility of a patient's foot.

As used herein, the "first ray position" (sometimes referred to as "first ray elevation") of a patient's foot 126 refers to a measurement of static displacement of the first ray from a weightbearing position. Specifically, the first ray position refers to a measurement of the plantar first metatarsal head height compared to the second metatarsal head while the patient is in a weightbearing position. As discussed in further detail below, the first ray position can be measured from a plantar aspect of the first metatarsal head upon application of a vertical load to the first metatarsal head. The "first ray position index" refers to a normalized version of the first ray position measurement. For example, if the first ray position measurement is clinically interpreted as a "raw" measurement, the first ray position index can be a normalized value, *e.g.,* a value that is determined based on a length measurement of the patient's foot (such as a truncated foot length or a total foot length, as described in further detail below), so as to provide standardized measurements and assessments that have utility for a wide variety of foot sizes.

The "first ray mobility" of a patient's foot 126 refers to a measurement of dynamic displacement of the first ray from a resting (*e.g.,* non-weightbearing) position. Specifically, the first ray mobility refers to a measurement of the change in dorsal first metatarsal head height occurring due to a load force applied to the first metatarsal head while the patient is in a non-weightbearing position. As discussed in further detail below, the first ray mobility can be measured from a dorsal aspect of the first metatarsal head upon application of a vertical load to the first metatarsal head. The "first ray mobility index" refers to a normalized version of the first ray mobility measurement. For example, if the first ray mobility measurement is clinically interpreted as a "raw" measurement, the first ray mobility index can be a normalized value, *e.g.,* a value that is determined based on a length measurement of the patient's foot (such as a truncated foot length or a total foot length, as described in further detail below), so as to provide standardized measurements and assessments that have utility for a wide variety of foot sizes.

The "arch height index" of a patient's foot 126 refers to a ratio of an arch height of the foot to a foot length. The "arch height" of the foot refers to a measurement of a height of the dorsum of the foot to the ground, measured at about one-half of the total foot length, *e.g.,* near the approximate apex of the arch. The foot length can be a measurement of truncated foot length or total foot length, as described in further detail below.

The "arch height flexibility" of a patient's foot 126 refers to a measurement of the change in the arch height of the foot from sitting to standing or non-weightbearing to weightbearing positions due to the change in load borne by the arch during these activities.

The "arch stiffness" of a patient's foot 126 refers to a measurement of deformation of the arch of the foot per unit of load. For example, one measure of arch stiffness can reflect the change in arch height index of the foot due to the increase in load from sitting to standing or non-weightbearing to weightbearing positions.

The "first MTP joint flexibility" of a patient's foot 126 refers to a measurement of flexion, such as dorsiflexion, in the first metatarsophalangeal joint (sometimes referred to as "MTPJ").

Reference will now be made in detail to the various exemplary embodiments of the subject disclosure illustrated in the accompanying drawings. Wherever possible, the same or like reference numbers will be used throughout the drawings to refer to the same or like features. It should be noted that the drawings are in simplified form and are not drawn to precise scale. Certain terminology is used in the following description for convenience only and is not limiting. Directional terms such as top, bottom, left, right, above, below and diagonal, are used with respect to the accompanying drawings. The term "anterior" refers to a direction towards a front of a body. The term "posterior" refers to a direction towards a back of the body and/or away from the "anterior" end. The term "medial" refers to a direction closer towards a midline of the body. The term "lateral" refers to a direction away from the midline of the body and/or away from the "medial" end. The term "superior" refers to a direction towards an upper, or "head," end of the body. The term "inferior" refers to a direction towards a lower end of the body and/or away from the "superior" end. The words "inwardly" and "outwardly" refer to directions toward and away from, respectively, the geometric center of the identified element and designated parts thereof. Such directional terms used in conjunction with the following description of the drawings should not be construed to limit the scope of the subject disclosure in any manner not explicitly set forth. Additionally, the term "a," as used in the specification, means "at least one." The terminology includes the words above specifically mentioned, derivatives thereof, and words of similar import.

"About" as used herein when referring to a measurable value such as an amount, a temporal duration, and the like, is meant to encompass variations of ±20%, ±10%, ±5%, ±1%, or ±0.1% from the specified value, as such variations are appropriate.

Throughout this disclosure, various aspects of the subject disclosure can be presented in a range format. It should be understood that the description in range format is merely for convenience and brevity and should not be construed as an inflexible limitation on the scope of the subject disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Furthermore, the described features, advantages and characteristics of the exemplary embodiments of the subject disclosure may be combined in any suitable manner in one or more embodiments.

Referring to FIGS. 1-3, the foot structure and function assessment device 100 includes a base 102, a clamp 106 attached to the base, a vertical measurement mechanism 128 carried by the base, an actuation member 202, and a controller 206 operatively in communication with the actuation member. While FIGS. 1-3 illustrate the device as applied to a right foot 126, the device can equally be configured for a left foot, such as by a mirrored construction of the device to accommodate the left foot.

The base 102 of the device 100 has a top surface 104. The top surface is a substantially planar surface sized sufficiently for supporting a patient's foot 126 by acting as a platform. The base includes an upper and a lower base plate, and four side panels defining a housing having a hollow interior. Thus, the base can house electronic components and circuitry within the device.

The base 102 also carries a positioning block 124 at an anterior end of the device for placing a first metatarsal head of the patient's foot 126 to facilitate measurements such as total foot length and truncated foot length, as described in further detail below. The positioning block includes a delve, slot, or hollow 136 that extends in a medial-lateral direction or widthwise direction of the base for mating or engaging with a base 142 of a clamp 106 to allow for adjustable and repeatable positioning of the clamp relative to a second metatarsal of the patient's foot. The longitudinal length of the delve extends sufficiently to allow the clamp to move from a medial end to a lateral end of the patient's foot. The delve can have an inverted-T shape that allows a clamp mount 140 of a clamp assembly 144 of the device to translate along the medial-lateral direction.

The foot structure and function assessment device 100 can optionally include a carrying handle 122. The carrying handle is secured to the base, *e.g.,* about its anterior-facing end, and facilitates transporting the device.

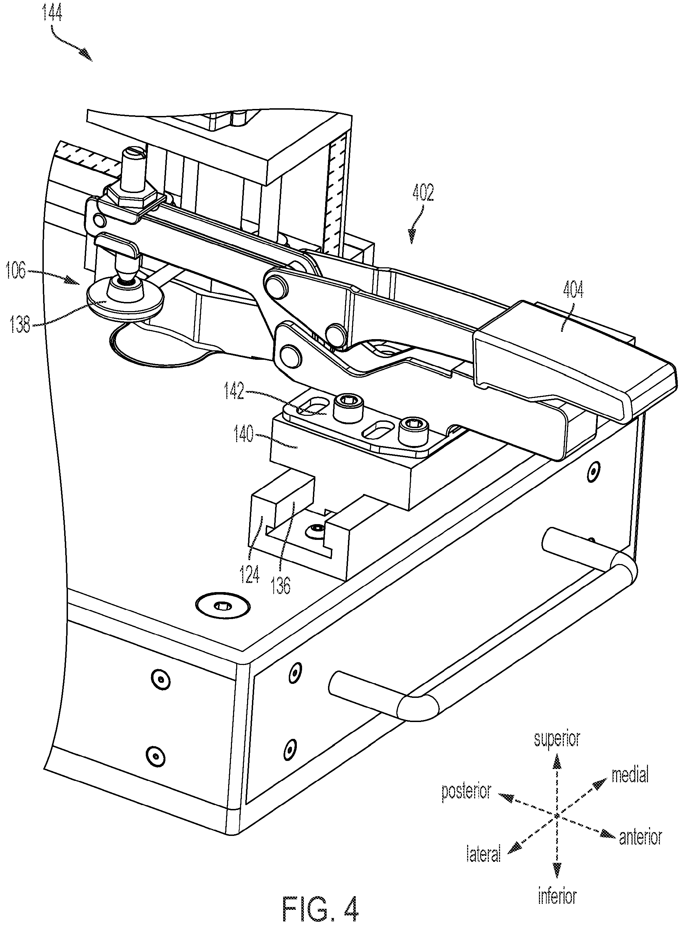

The clamp 106 is structured as best shown in FIG. 4 and is attached via a clamp assembly 144 to the base 102 of the device 100. The clamp is for immobilizing a second metatarsal of the patient's foot 126. The clamp is operable to mechanically ground to the device the second metatarsal head of the foot, *e.g.,* by applying a clamping force of about 110 N to the dorsal surface of the second metatarsal. The clamp is an auto-adjustable toggle clamp. Specifically, the clamp includes a horizontal toggle linkage 402 operable to apply an auto-adjustable clamping force. The clamp includes an open arm and a horizontal base plate. A head 138 of the clamp is coupled to the open arm. The head has a substantially planar or flat surface at the ventral surface of its distal end for interfacing with the dorsal surface of the second metatarsal of the patient's foot. The clamp is movably connected to the base of the device and includes a quick release handle 404. The quick release handle allows the patient or a user of the device to remove clamping force from the patient's foot at any time during foot structure and function assessment, or relieve any perception of undesired force on the foot. The clamp is laterally spaced from an actuation member 202 operable for vertically moving the first ray. In other words, the clamp is spaced from the actuation member such that the clamp spatially corresponds to and is operable to immobilize the second metatarsal of the foot as the actuation member vertically translates the first ray. Specifically, the head of the clamp is laterally spaced from the actuation member.

As best seen in FIG. 4, the clamp assembly 144 includes a mount 140 that is rigidly connected to a base 142 of the clamp 106. The clamp assembly is horizontally adjustable in a widthwise direction along the base of the device (*e.g.,* in the medial-lateral direction). Specifically, the mount is matingly engaged with the delve 136 of the positioning block 124 and movable along the delve in the medial-lateral direction to allow for adjusting a position of the clamp relative to the second metatarsal. The mount can include laterally protruding ribs or flanges along the anterior and posterior ends that matingly engage with the inverted-T shape of the delve of the positioning block. The mount allows the clamp to be adjustable along a medial-lateral axis of the foot so as to allow for widthwise or medial-lateral translation relative to the foot and accommodate different foot widths.

Referring back to FIG. 1, the vertical measurement mechanism 128 is carried by the base 102 of the device 100. The vertical measurement mechanism is for measuring displacement of a first ray of the patient's foot from its normal resting position. The vertical measurement mechanism can be a vertical graticule that is coupled to a block adjacent an actuator 114 and secured to the base 102. The vertical graticule can be delineated with graduations, *e.g.,* millimeter and/or centimeter markings.

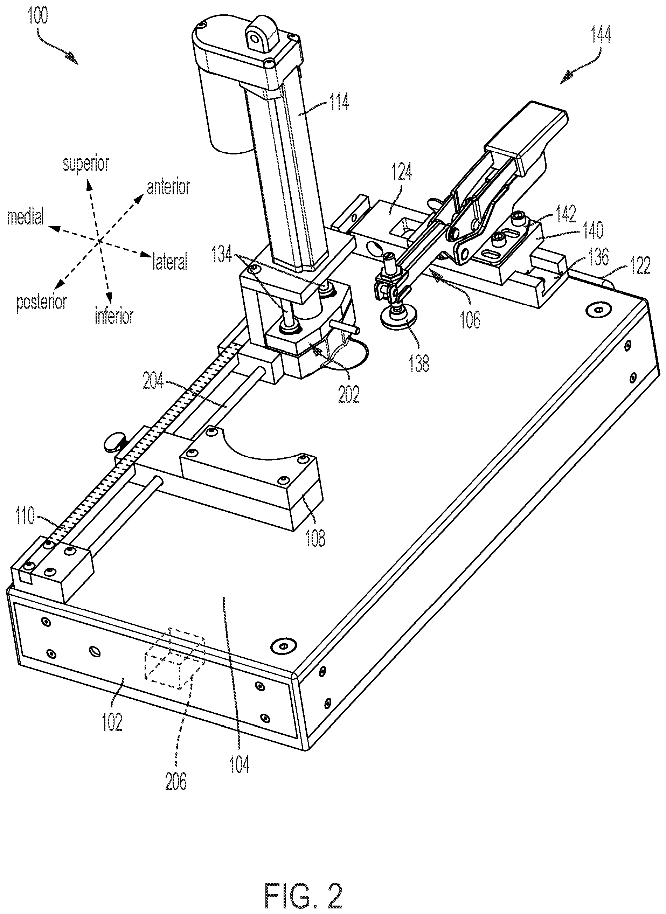

As best illustrated in FIGS. 2, 3, and 5, the actuation member 202 of the device 100 includes an actuator 114 and a block assembly 538. The actuation member is operable for vertically displacing the first ray of the patient's foot 126 (*e.g.,* in the superior-inferior direction) from its normal rest position.

The actuator 114 can be a linear actuator, cylinder, servomotor, and the like. A negative feedback servo controls a force set point of the actuator 114. The negative feedback servo can use a load cell 530 and an amplifier in connection with the actuator, as described in further detail below. In operation, as the actuator applies a vertical load (*e.g.,* a first vertical load or a second vertical load) to a load cell assembly 520, the load cell assembly is coupled to a shaft of the actuator and displaces a lower block 504 of the block assembly 538 in the superior-inferior direction. In turn, the lower block can displace the first ray of the patient's foot in the superior-inferior direction from its rest position. When the lower block displaces the first ray with a sufficient vertical load as measured by the load cell 530, a dorsal surface of the first metatarsal head of the patient's foot translates the rod 510 of the upper block 502 of the actuation member in the superior direction.

The actuator is operable to apply a first vertical load of about 5 N to the first ray. The first vertical load can range from about 3 N to about 10 N, including about 3, 4, 5, 6, 7, 8, 9, and 10 N or more. The first vertical load is associated with assessment of a first ray position of the patient's foot. Specifically, application of the first vertical load by the actuator results in a corresponding vertical translation of the lower block 504 of the actuation member. A user of the device 100 can then use the vertical measurement mechanism 128 to measure a first ray position of the patient's foot, for example, by measuring a position of a first movable pointer 130 of the lower block relative to the vertical measurement mechanism. In other words, the first vertical load applied to the first ray defines the first ray position.

The actuator is also operable to apply a second vertical load of about 50 N to the first ray. The second vertical load can range from about 45 N to about 55 N, including about 45, 46, 47, 48, 49, 50, 51, 52, 53, 54, and 55 N or more. The second vertical load is associated with assessment of a first ray mobility of the patient's foot. Specifically, application of the second vertical load by the actuator results in a corresponding vertical translation of the upper block 502 of the actuation member. A user of the device 100 can then use the vertical measurement mechanism 128 to measure a first ray mobility of the patient's foot, for example, by measuring the second movable pointer 132 of the upper block relative to the vertical measurement mechanism. In other words, the second vertical load applied to the first ray defines the first ray mobility.

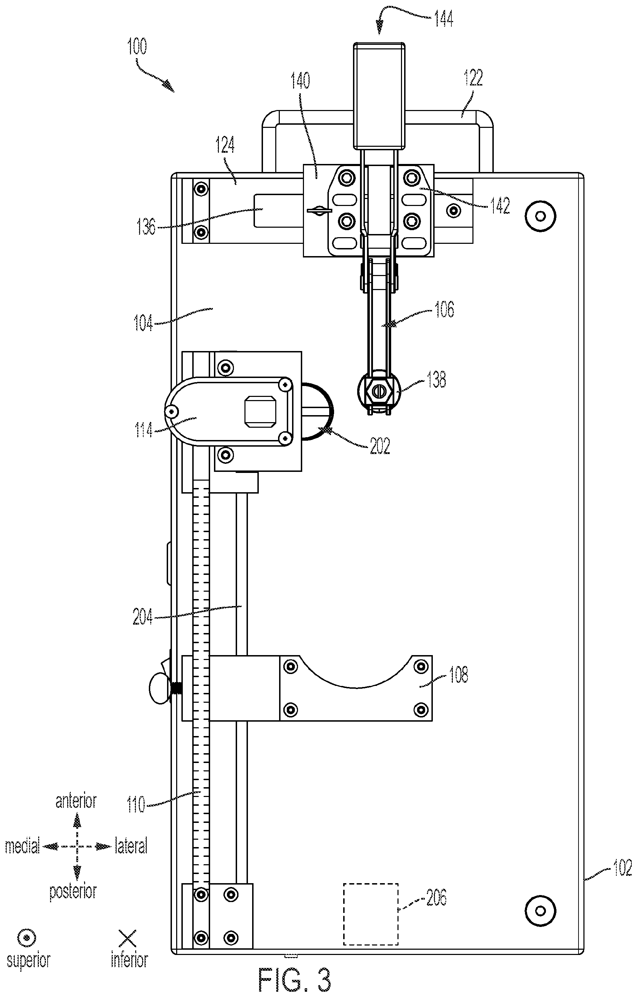

The block assembly 538 includes an upper block 502 and a lower block 504. The upper and lower blocks can be physically separate. The block assembly includes a central through hole 514 for mating engagement with the actuator 114 (FIGS. 2, 3). A shaft of the actuator extends through the central through hole 514 and is connected to the lower block 504. The actuation member also includes a pair of vertical guide rods 134 coupled to the base for guiding movement of the block assembly in the vertical direction (*e.g.,* the superior-inferior direction). The guide rods 134 extend through respective through holes 514 in the block assembly. Linear bearings 516 are provided on the guide rods for frictionless superior-inferior translation along the guide rods.

The lower block 504 of the actuation member 202 includes a platform 506 structured to contact a plantar surface of the first ray (*e.g.,* a lower surface). The upper block includes a rod 510 extending laterally relative to the actuator 114. In operation, the patient inserts the foot such that the first metatarsal is positioned between the platform on the lower block and the rod on the upper block of the block assembly. The lower block includes a concave recess 518 structured to accommodate a medial side of the patient's foot. The lower block also includes a pillar 508 that engages with a load cell assembly 520 positioned beneath the pillar. The load cell 530 measures a vertical load applied by the actuator 114 that is operable to vertically translate the lower block of the actuation member (*e.g.,* in the superior-inferior direction).

The actuation member 202 also includes a first movable pointer 130 and a second movable pointer 132 adjacent the vertical measurement mechanism 128. The first movable pointer is on the lower block 504 of the block assembly. The first movable pointer is for measuring a first ray position of the patient's foot. For example, in operation a user can visually identify a first measurement on the vertical measurement mechanism 128 based on the position of the first movable pointer after the actuator 114 has applied a first vertical load to the first ray (*e.g.,* of about 5 N). The second movable pointer is on the upper block 502 of the block assembly. The second movable pointer is for measuring a first ray mobility of the patient's foot. For example, in operation a user can visually identify a second measurement on the vertical measurement mechanism based on the position of the second movable pointer after the actuator has applied a second vertical load to the first ray (*e.g.,* of about 50 N).

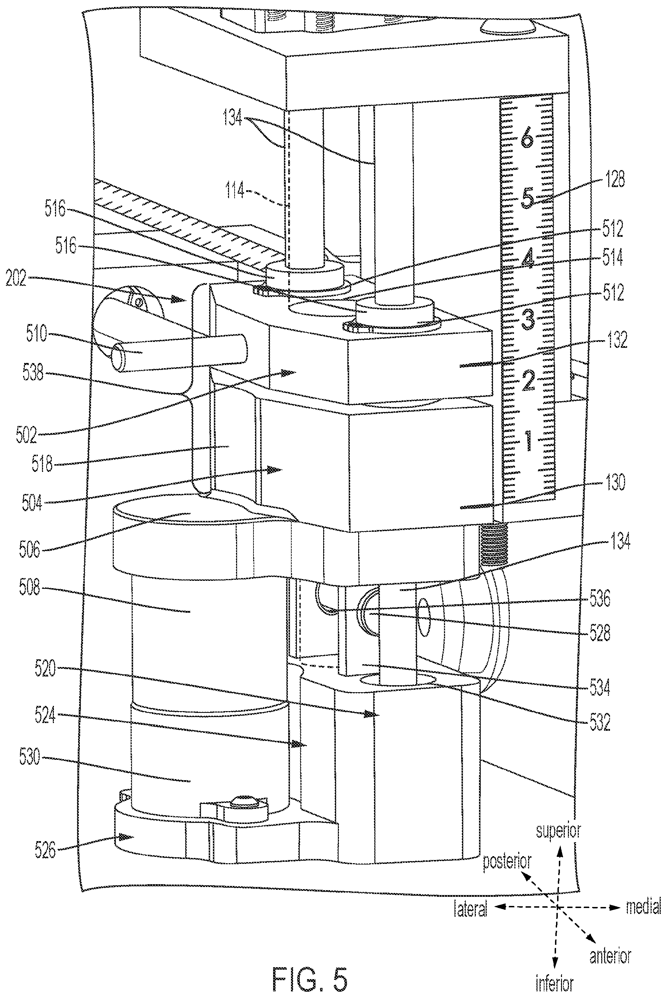

As best illustrated in FIG. 5, a load cell assembly 520 of the device 100 is coupled to a shaft of the actuator 114 and includes a platform 524 and a block 526. The platform has load cell 530 mounted to a dorsal surface of the platform. The load cell is operable to measure the force applied by the actuator against the pillar 508 of the actuation member 202.

The block 526 of the load cell assembly 520 includes a pair of prongs 534. The prongs extend proud from a dorsal surface of the block. The prongs each include a through hole 536. A fastener 528 extends through each through hole and through the shaft of the actuator so as to couple the load cell assembly to the shaft of the actuator. The block includes a pair of through holes 532 for receiving the vertical guide rods 134 from the actuation member 202 for guiding movement of the load cell assembly in the vertical direction (*e.g.,* the superior-inferior direction). The load cell assembly also includes a pair of linear bearings, one in each through hole, for frictionless translation in the superior-inferior direction.

Referring back to FIGS. 2 and 3, a controller 206 of the device 100 is operatively in communication with the actuation member 202. The controller is housed in the hollow interior of the housing of the base 102. The controller is configured to displace the first ray from its normal resting position while the remainder of the patient's foot is immobilized in its normal resting position, by applying a first and second vertical load. The controller can control the force servo of the actuator 114 for assessment of a first ray position and mobility of a patient's foot. The controller can be a programmable logic controller, a microcontroller, a computer, and the like.

Referring back to FIGS. 1-3, the device 100 includes a heel cup 108 attached to the base 102 of the device and movable relative thereto, and a horizontal measurement mechanism 110 carried by the base. The heel cup includes a concave recess structured to accommodate the patient's rearfoot. The heel cup is coupled to a slide track 204 (FIGS. 2, 3) and slidably adjustable in the anterior-posterior directions. In operation, the user of the device inserts the patient's rearfoot into the heel cup which positions a first metatarsal head (*e.g.,* the "big toe") of the patient's foot against the positioning block 124 coupled to the base. Advantageously, the heel cup may also be modified to permit mechanically grounded fixation of the rearfoot in positions useful for assessment of foot structure and function, such as resting calcaneal stance position, neutral calcaneal stance position, etc. The user can then use the horizontal measurement mechanism to quantify foot length, to facilitate normalizing the foot structure and function assessments as described in further detail below.

The horizontal measurement mechanism 110 is for quantifying foot length. The horizontal measurement mechanism can be a horizontal graticule that is secured to the base. The horizontal graticule can be delineated with graduations, *e.g.,* millimeter and/or centimeter markings. Advantageously, in operation the horizontal measurement mechanism facilitates foot length measurements that allow for normalizing foot structure and function assessments including first ray position, first ray mobility, arch height, for example, to determine a first ray position index, a first ray mobility index, or an arch height index, respectively, of the patient's foot. By way of non-limiting example, the foot length can include truncated foot length and total foot length. As used herein, "truncated foot length" refers to measurement of a length of the foot stopping at (*e.g.,* without measuring) the phalanges. "Total foot length" (sometimes referred to as maximal foot length) refers to measurement of a length of the entire foot, including the phalanges.

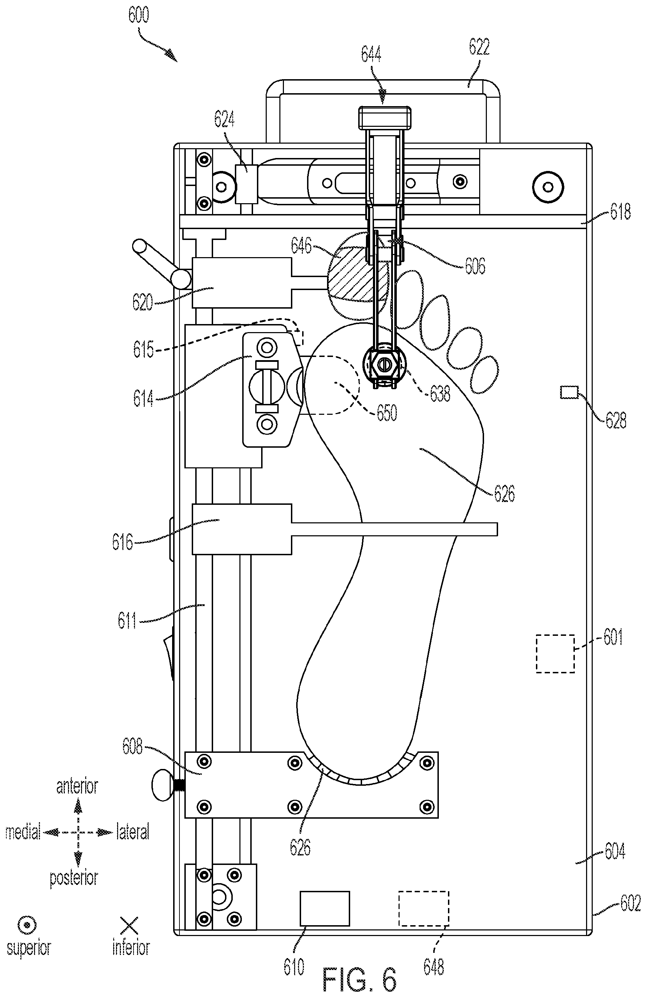

FIG. 6 illustrates another exemplary embodiment of a foot structure and function assessment device 600 in accordance with the subject disclosure. This exemplary embodiment of the foot structure and function assessment device operates and includes features substantially as disclosed for the above embodiment, except as specifically discussed hereinafter. The present exemplary embodiment includes an arch height measurement mechanism 616, a foot length measurement mechanism 618, and a torque member 620. While FIG. 6 illustrates the device as applied to a right foot 626, the device can equally be configured for a left foot, such as by a mirrored construction of the device to accommodate the left foot.

The base 602 of the device 600 includes a top surface 604 for supporting the patient's foot 626. The base is generally configured as discussed above for the base 102. Additionally, the base 602 can include a scale 601 for measuring a body weight of the patient. Advantageously, the body weight can be used to determine and assess an arch height flexibility and an arch stiffness of the patient's foot, as described in further detail below.

A clamp 606 is movably attached to the base 602 of the device 600. For example, the clamp 606 can be attached via a slidable clamp assembly 644 to a positioning block 624 rigidly secured to the base of the device so that the clamp assembly is horizontally adjustable in a widthwise direction along the base of the device (*e.g.,* in the medial-lateral direction). The clamp is generally configured as discussed above for the clamp 106. However, instead of a planar or flat ventral surface of the head of the clamp at the interface between the dorsal surface of the second metatarsal of the patient's foot, the clamp 606 can optionally include a head 638 having a concave ventral surface 639 at the interface with the dorsal surface of the second metatarsal. The concave surface allows for an improved fit with the second metatarsal so as to increase overall reliability and consistency of the foot structure and function assessments.

The horizontal measurement mechanism and the vertical measurement mechanism are carried by the base 602 of the device 600. Instead of a horizontal and vertical graticule as used with the horizontal and vertical measurement mechanisms 110, 128 of the device 100 (FIG. 1), the device 600 includes a horizontal position sensor 610 and a vertical position sensor 628 operatively in communication with a controller 648. The output of the position sensors 610, 628 is used for automatic measurement of foot structure and function including the foot length, first ray mobility, and first ray position of the patient's foot. The position sensors can be, *e.g.,* linear variable differential transformers (LVDTs). The position sensors increase overall reliability and consistency of the measurements of foot length, first ray mobility, and first ray position, for example, by avoiding potential visual errors associated with visual artifacts such as parallax and manual comparison of a first or second movable pointer 130, 132 with a measurement graduation on a vertical or horizontal graticule 128, 110.

A foot length measurement mechanism 618 is movably attached to the base 602 of the device 600 and extends in a widthwise direction (*e.g.,* a medial-lateral direction) of the base. The foot length measurement mechanism can be a bar spanning a width of the device transverse to a slide track 611 rigidly coupled about a lateral side of the base. The foot length measurement mechanism is coupled to the slide track and slidably adjustable in the anterior-posterior direction along the slide track. The foot length measurement mechanism allows for measuring a total foot length of the patient's foot. The total foot length can be used to normalize assessments of foot structure and function including the first ray mobility, first ray position, arch height index, arch height flexibility, and/or arch stiffness of the patient's foot, as described in further detail below.

An actuator 614 is carried by the base 602 of the device 600. The actuator is coupled to an actuation member 650 operable for vertically displacing the first ray of the patient's foot. The actuation member is generally configured as discussed above for the actuation member 202 of the device 100. Additionally, the actuation member of the present exemplary embodiment includes a position sensor 615 operatively in communication with the controller 648 whose output can be used to determine, *e.g.,* in the anterior-posterior direction, a truncated foot length of the foot. The truncated foot length can be used to determine assessments of foot structure and function including the first ray position index, first ray mobility index, and arch height index, as described in further detail below.

A heel cup 608 is movably attached to the base 602 of the device 600. The heel cup is coupled to the slide track 611 and slidably adjustable in the anterior-posterior directions along the slide track. The heel cup is generally configured as discussed above for the heel cup 108. Additionally, a lining 626 is provided in a concave recess of the heel cup 608. The lining can be formed of a material selected to be compliant with (*e.g.,* conforming to) the patient's rearfoot. For example, the lining can be composed of a conforming silicone or fabric. The compliant lining allows for an improved fit with the patient's rearfoot so as to increase overall reliability and consistency of the foot structure and function assessments.

An arch height measurement mechanism 616 is movably attached to the base 602 of the device 600. The arch height measurement mechanism is coupled to the slide track 611 and slidably adjustable in the anterior-posterior directions along the slide track. The arch height measurement mechanism includes a bar coupled to a vertical pillar for being slidably adjustable in the superior-inferior directions. The bar can optionally include a lining selected to be compliant with (*e.g.,* conforming to) the dorsal surface of the patient's foot, for an improved fit to increase overall reliability and consistency of the foot structure and function assessments.

The arch height measurement mechanism is operable for measurement of the arch height of the patient's foot. The arch height can be used to determine and assess an arch height index, an arch height flexibility, and an arch stiffness of the patient's foot. In operation, the user can move the arch height measurement mechanism, *e.g.,* along the anterior-posterior direction, to position the arch height measurement mechanism along a length of the patient's foot (*e.g.,* at a location at about one-half of the patient's total foot length) as described in further detail below. The user can then rest the arch height measurement mechanism atop a surface (*e.g.,* a dorsal surface) of the patient's foot. The corresponding height of the patient's foot can then be measured, *e.g.,* using the vertical measurement mechanism of the device such as an LVDT, a position sensor, or a vertical graticule, to determine and assess the arch height index, arch height flexibility, and arch stiffness of the foot.

A torque member 620 is movably attached to the base 602 of the device 600. Specifically, the torque member is coupled to the slide track 610 and slidably adjustable in the anterior-posterior directions along the slide track so as to position the torque member near the location of the first metatarsal head. The torque member is attachable to a first metatarsal head (*e.g.,* the "big toe") of a patient's foot. The torque member can include a fastener 646 that circumscribes the first metatarsal head, such as a hook-and-loop fastener. In operation, a user can use the torque member to apply a torque force so as to rotate the first metatarsal head up or down throughout a range of motion. Specifically, the torque member is operable to apply a moment about an axis of rotation of a first MTP joint (*e.g.,* the axis of plantarflexion-dorsiflexion) of the foot. Alternatively, a controller 648 of the device can be operatively in communication with the torque member to apply the joint moment throughout the range of motion of the first MTP joint. The torque member can be used to determine and assess a first MTP joint flexibility of the foot. Specifically, the controller is operable to determine and assess a first MTP joint flexibility of the patient's foot based on the applied moment and angular excursion that are measured from the torque member throughout the range of motion of the first MTP joint, *e.g.,* using electrical output from a variable resistor and/or torque transducer that is coupled to the controller.

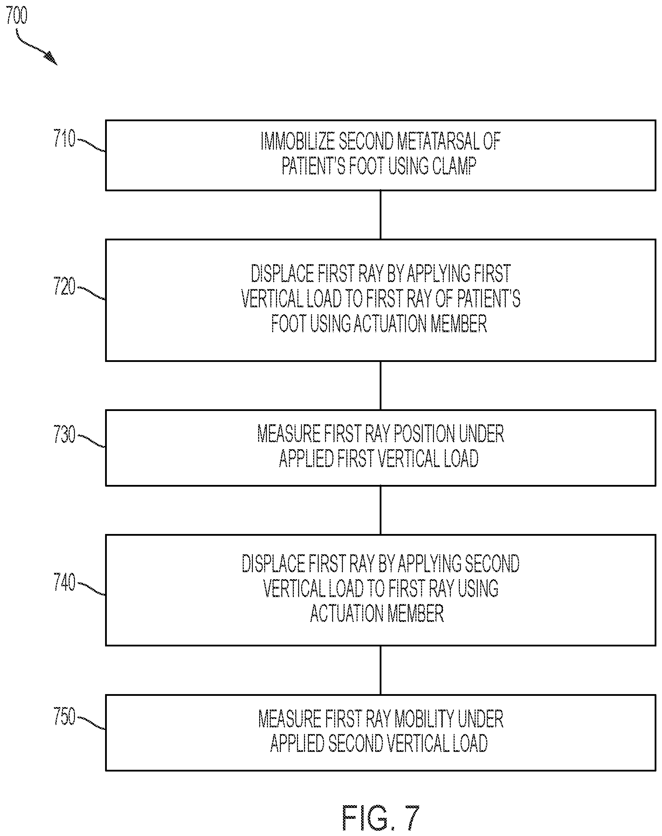

In operation, referring to FIGS. 1-6, a user places a rearfoot of a patient's foot into the heel cup and a first metatarsal head (*e.g.,* the "big toe") of the foot against the positioning block coupled to the base of the device 100, 600. The heel of the foot is unconstrained. Advantageously, the structure of the device thereby provides a choice for the user to place the foot either in subtalar joint neutral (STJN) or resting calcaneal stance position (RCSP). The user immobilizes the second metatarsal of the patient's foot using the clamp, and operates the controller so as to use the actuation member to apply a first and a second vertical load to the first ray, so as to assess a first ray position and mobility of the patient's foot, as discussed below in further detail in connection with FIG. 7.

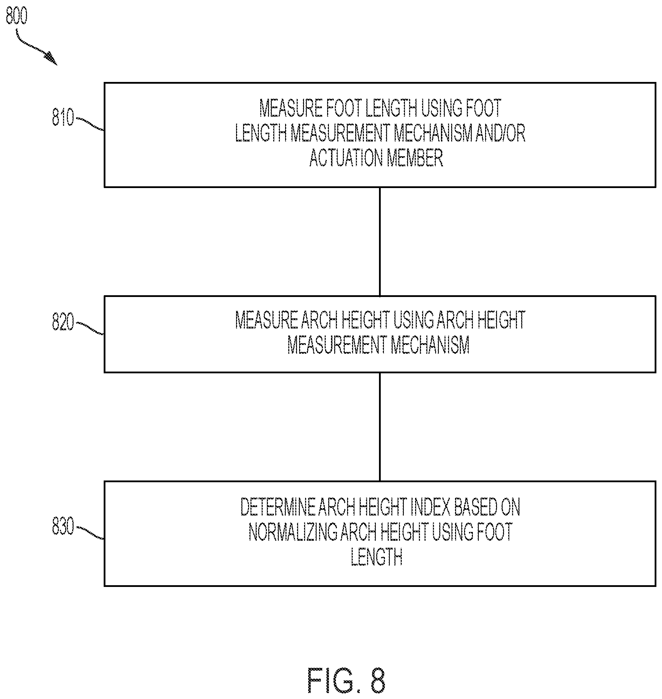

Optionally, the user slides the foot length measurement mechanism in the anterior-posterior directions to the appropriate position relative to the patient's foot so as to measure the foot length of the patient's foot (*e.g.,* either the truncated foot length or the total foot length). The user slides the arch height measurement mechanism in the anterior-posterior directions to a position located at about one-half of the patient's total foot length, and slides the arch height measurement mechanism in the inferior direction to rest the arch height measurement mechanism at a position located atop a dorsal surface of the patient's foot, so as to measure an arch height of the patient's foot. The user determines the arch height index of the patient's foot based on normalizing the measured arch height by the measured foot length, as discussed below in further detail in connection with FIG. 8.

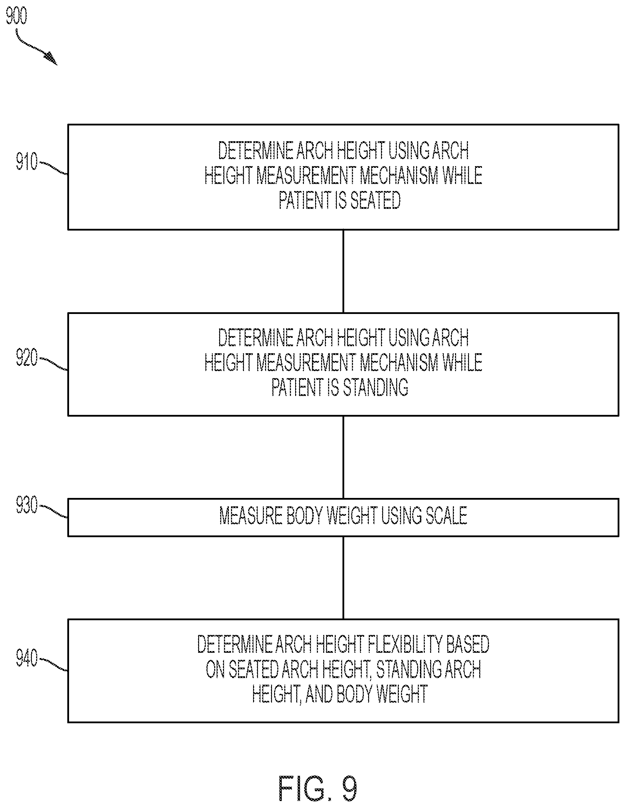

Optionally, the user uses the arch height measurement mechanism to determine an arch height of the patient's foot while the patient is seated, and an arch height of the patient's foot while the patient is standing. The user further measures the patient's body weight using the scale. The user then determines the arch height flexibility of the patient's foot based on the seated arch height, the standing arch height, and the measured body weight, as described below in further detail in connection with FIG. 9.

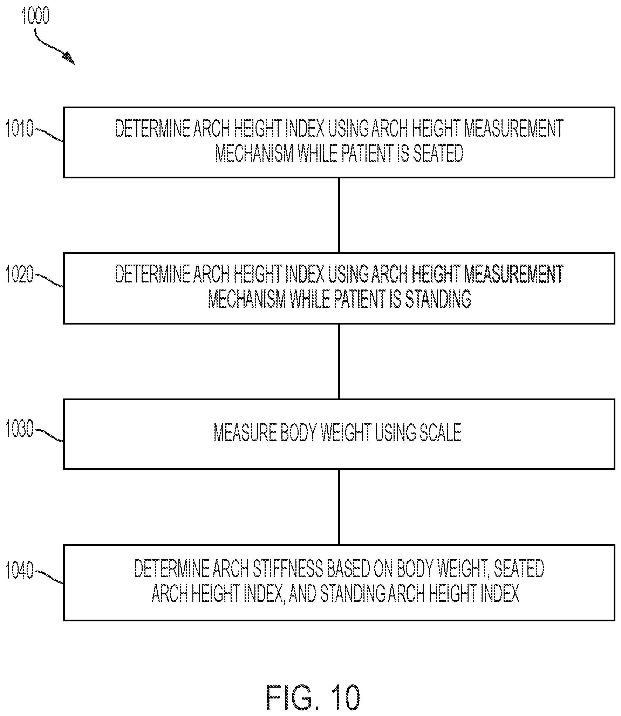

Optionally, the user uses the arch height measurement mechanism to determine an arch height index of the patient's foot while the patient is seated, and an arch height index of the patient's foot while the patient is standing. The user further measures the patient's body weight using the scale. The user then determines the arch stiffness of the patient's foot based on the measured body weight, the seated arch height index, and the standing arch height index, as described below in further detail in connection with FIG. 10.

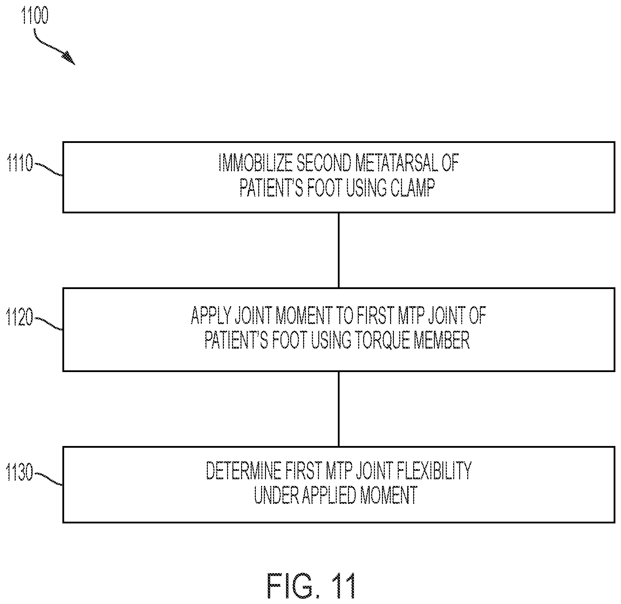

Optionally, the user immobilizes the second metatarsal of the patient's foot using the clamp, and uses the torque member to apply a torque force to the first MTP joint, so as to rotate the first metatarsal head throughout a range of motion. A controller operatively in communication with the torque member (*e.g.,* via a variable resistor and/or torque transducer) can measure the accompanying joint moment and the accompanying angular excursion applied throughout the range of motion to determine the first MTP joint flexibility of the patient's foot, as discussed below in further detail in connection with FIG. 11.

Referring to FIG. 7, a flowchart is shown of an exemplary method 700 for assessing foot structure and function including a first ray mobility and position of the patient's foot using the foot structure and function assessment devices of the subject disclosure.

The foot structure and function assessment device immobilizes a second metatarsal of the patient's foot against the base (step 710). For example, the user grounds the second metatarsal head using the clamp. The user can place the patient in STJN or RCSP during examination. The clamp can be applied with about 110 N of force or a sufficient amount of force to immobilize the second metatarsal. Should the clamp need to be released, a quick release handle may be toggled to remove the clamping force from the patient's foot at any time during examination.

The first ray of the patient's foot is displaced by applying a first vertical load (step 720). The actuation member can apply an exemplary first vertical load of about 3 N to about 10 N, and preferably about 5 N.

The first ray position of the patient's foot under the applied first vertical load is then measured (step 730). The measurement is assessed via a first movable pointer of an actuation member adjacent a vertical measurement mechanism of the device under the applied first vertical load. Alternatively, the device can determine a measurement and assessment of first ray position automatically using the vertical measurement mechanism of the device under the applied first vertical load, such as by using the output from an LVDT or other position sensor to identify the position of the first metatarsal head of the foot.

The method 700 further includes determining a first ray position index of the patient's foot based on normalizing the first ray mobility using a foot length of the patient's foot. Specifically, the user can determine a truncated foot length of the foot using a horizontal measurement mechanism of the device or output from a position sensor of an actuation member of the device, as described in further detail above. The first ray position index of the foot is determined by dividing the first ray position by the truncated foot length. Alternatively, the user may determine the total foot length of the foot using the horizontal measurement mechanism of the device or the foot length measurement mechanism of the device, as described in further detail above. The first ray position index is then determined by dividing the first ray position by the total foot length.

The device further displaces the first ray by applying a second vertical load to the first ray (step 740). The actuator of the device can apply a second vertical load from about 45 N to about 55 N, and preferably about 50 N to the first ray.

The device measures a first ray mobility of the patient's foot under the applied second vertical load (step 750). That is, the user determines a measurement and assessment of the first ray mobility of the foot based on the position of the second movable pointer of the actuation member adjacent the vertical measurement mechanism of the device. Alternatively, the device determines a measurement and assessment of the first ray mobility automatically using a vertical measurement mechanism of the device, such as by using the output from an LVDT or other position sensor to identify a position of the first metatarsal head of the foot.

The method 700 further includes determining a first ray mobility index of the patient's foot based on normalizing the first ray mobility using a foot length of the foot. Specifically, the user may determine the truncated foot length of the foot using the horizontal measurement mechanism of the device or output from a position sensor of an actuation member of the device, as described in further detail above. The first ray mobility index of the foot may be determined by dividing the first ray mobility by the truncated foot length. Alternatively, the user may determine the total foot length of the foot using the horizontal measurement mechanism of the device or the foot length measurement mechanism of the device, as described in further detail above. The first ray mobility index may be then determined by dividing the first ray mobility by the total foot length.

The method 700 further includes applying one or more initial vertical loads prior to applying the first and second vertical loads. Advantageously, application of one or more initial loads prior to applying the first and second vertical loads assists in controlling for (*e.g.,* reducing an undesired effect of) recent strain history on the first ray of the patient's foot. For example, the controller can be configured to use the actuation member 202 (FIGS. 2, 3, 5) to apply initial loads of about ten cyclic loads of about 25 N to control for recent strain history of the soft tissues of the first ray. Alternatively, the controller and the actuation member can apply 1, 2, 3, 4, 5, 6, 7, 8, 9, or more than 10 initial cyclic loads, as beneficial to control for recent strain history. The load force applied by the initial cyclic loads can be from about 20 N to about 50 N.

Referring to FIG. 8, a flowchart is shown of an exemplary method 800 for assessing foot structure and function including an arch height index of the patient's foot using the foot structure and function assessment device of the subject disclosure.

The device measures a foot length of a patient's foot (step 810). Specifically, the user can measure a truncated foot length of the foot automatically using output from a position sensor of the actuation member of the device, as described in further detail above. Alternatively, the user can measure a total foot length of the foot using the foot length measurement mechanism of the device, as described in further detail above.

The device determines an arch height of the foot via the arch height measurement mechanism (step 820). The arch height measurement mechanism is moved along the anterior-posterior direction about a length of the patient's foot, *e.g.,* to a position located at about one-half the patient's total foot length. The user lowers the arch height measurement mechanism in the superior-inferior direction onto a dorsal surface of the foot. The device then determines a measurement of arch height based on the position of the arch height measurement mechanism. The arch height can be determined while the patient is seated or standing, or non-weightbearing or weightbearing.

The device determines an arch height index of the patient's foot based on normalizing the arch height using a foot length of the patient's foot (step 830). Specifically, the arch height index is determined by dividing the arch height by the truncated foot length. Alternatively, the arch height index is determined by dividing the arch height by the total foot length. The arch height index can be determined while the patient is seated or standing.

Referring to FIG. 9, a flowchart is shown of an exemplary method 900 for assessing foot structure and function including an arch height flexibility of the patient's foot using the foot structure and function assessment device of the subject disclosure.

An arch height of a patient's foot is determined while the patient is seated or non-weightbearing (step 910). The user positions the arch height measurement mechanism along the anterior-posterior direction about a length of the patient's foot, *e.g.,* at a location at about one-half the patient's total foot length. The user lowers the arch height measurement mechanism along the superior-inferior direction onto a dorsal surface of the foot. The device then determines a measurement of seated arch height ($AH_{seated}$) based on the position of the arch height measurement mechanism.

Then an arch height of the foot is determined while the patient is standing or weightbearing (step 920). The user positions the arch height measurement mechanism along the anterior-posterior direction about a length of the patient's foot, *e.g.,* at a location at about one-half the patient's total foot length. The user lowers the arch height measurement mechanism along the inferior direction onto the dorsal surface of the foot. The device then determines a measurement of standing arch height ($AH_{standing}$) based on the position of the arch height measurement mechanism.

The device then measures a body weight of the patient (step 930). For example, the patient's weight is measured via the weighing scale of the base of the device.

An arch height flexibility (AH Flexibility) is determined based on the seated arch height, the standing arch height, and the body weight (step 940). The arch height flexibility generally measures a change in the patient's arch height between sitting and standing, normalized to the change in load (*e.g.,* weight) on the foot. In some exemplary embodiments, the change in load may be approximated by about 40% of the body weight. The change in load was based on an assumed change in body weight from sitting to standing. The standing condition assumes the weight on the foot to be 50% of the body weight on each foot, and the sitting condition assumes the weight on the foot to be 10% of the body weight. Therefore, there was an assumed 40% change in load from standing to sitting (40% = 50% - 10%). Specifically, the arch height flexibility is determined by subtracting the standing arch height from the sitting arch height, dividing the result by the change in load, and normalizing the result. For example, the arch height flexibility can be determined by the following formula:

$$\text{AH Flexibility} = \frac{AH_{seated} - AH_{standing}}{40\% \times \text{Body weight}} \times 100$$

Referring to FIG. 10, a flowchart is shown of an exemplary method 1000 for assessing foot structure and function including an arch stiffness of the patient's foot using the foot structure and function assessment device of the subject disclosure.

An arch height index of a patient's foot is determined while the patient is non-weightbearing, *e.g.,* seated (step 1010). The user positions the arch height measurement mechanism along the anterior-posterior direction about a length of the patient's foot, *e.g.,* at a location at about one-half the patient's total foot length. The user lowers the arch height measurement mechanism along the superior-inferior direction onto a dorsal surface of the foot. The user then positions the foot length measurement mechanism along the anterior-posterior direction to the appropriate position relative to the patient's foot so as to measure the foot length of the patient's foot (*e.g.,* either the truncated foot length or the total foot length). The device then determines a measurement of seated arch height index ($AHI_{seated}$) based on the positions of the arch height measurement mechanism and the foot length measurement mechanism.

Then an arch height index of the foot is determined while the patient is weightbearing, *e.g.,* standing (step 1020). The user positions the arch height measurement mechanism along the anterior-posterior direction about a length of the patient's foot, *e.g.,* at a location at about one-half the patient's total foot length. The user lowers the arch height measurement mechanism along the inferior direction onto the dorsal surface of the foot. The user then positions the foot length measurement mechanism along the anterior-posterior direction to the appropriate position relative to the patient's foot so as to measure the foot length of the patient's foot (*e.g.,* either the truncated foot length or the total foot length). The device then determines a measurement of standing arch height index ($AHI_{standing}$) based on the positions of the arch height measurement mechanism and the foot length measurement mechanism.

The device then measures a body weight of the patient (step 1030). For example, the patient's weight is measured via the weighing scale of the base of the device.

An arch stiffness is determined based on the body weight, the seated arch height index, and the standing arch height index (step 1040). The arch stiffness generally measures deformation of the arch of the foot per unit of load (*e.g.,* weight) on the foot. In some exemplary embodiments, the change in load may be approximated by about 40% of the body weight. The change in load was based on an assumed change in body weight from sitting to standing. The standing condition assumes the weight on the foot to be about 50% of the body weight on each foot, and the sitting condition assumes the weight on the foot to be about 10% of the body weight. Therefore, there was an assumed about 40% change in load from standing to sitting (40% = 50% - 10%). Specifically, the arch stiffness is determined by subtracting the standing arch height index from the seated arch height index, and dividing the change in load by the result. For example, the arch stiffness can be determined by the following formula:

$$\text{Arch Stiffness} = \frac{40\% \times \text{Body weight}}{AHI_{seated} - AHI_{standing}}$$

Referring to FIG. 11, a flowchart is shown of an exemplary method 1100 for assessing foot structure and function including a first MTP joint flexibility of the patient's foot using the foot structure and assessment device of the subject disclosure.

The device immobilizes a second metatarsal of the patient's foot (step 1110). For example, the user grounds the second metatarsal head using the clamp. The user can place the patient in STJN or RCSP during examination. The clamp can apply sufficient force, *e.g.,* about 110 N of force, to immobilize the second metatarsal.

A joint moment is then applied to the first MTP joint of the foot (step 1120). For example, the user uses the torque member of the device to apply a joint moment to the first metatarsal head about the axis of plantarflexion-dorsiflexion (*e.g.,* rotate the big toe up and down). The device measures the moment applied throughout a range of motion of the first metatarsal head, for example via electrical output from a torque transducer such as a strain gauge-based extension socket torque transducer in communication with the controller. The device can also measure an amount of accompanying angular excursion about the axis of plantarflexion-dorsiflexion throughout the range of motion, for example via electrical output from a variable resistor such as a potentiometer coupled to the controller.

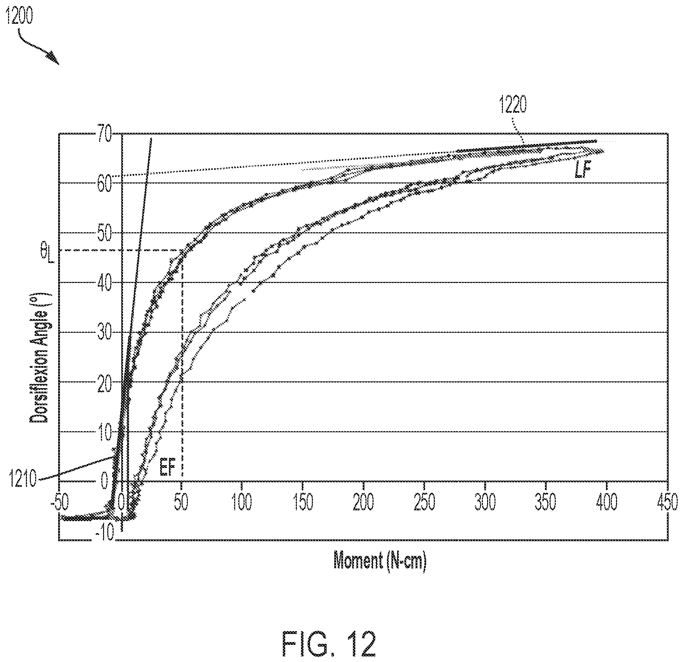

The first MTP joint flexibility under the applied joint moment is determined (step 1130). Referring to FIG. 12, the device can determine the first MTP joint flexibility by measuring the joint moment and dorsiflexion angle throughout a range of motion of the first metatarsal head of the patient's foot. An exemplary curve 1200 illustrates the moment plotted on the x-axis and the angle plotted on the y-axis. The first MTP joint flexibility of the patient's foot can generally be determined based on a slope of the curve. An "early" first MTP joint flexibility can be determined based on the slope 1210 of the curve during, for example, the initial 25% of the first MTP joint's range of motion. A "late" first MTP joint flexibility can be determined based on the slope 1220 of the curve during, for example, the final 25% of the first MTP joint's range of motion. Alternatively, the first MTP joint flexibility can be determined based on a computation of the laxity of the patient's first MTP joint, *e.g.,* by determining the angular displacement for a given or specified joint moment.

The various exemplary embodiments of the foot structure and function assessment device discussed herein provide numerous advantages over conventional foot structure and function assessment devices. For example, the present foot structure and function assessment device provides an actuation member and a vertical measurement mechanism, *e.g.,* actuation member 202, 650 and vertical measurement mechanism 128, 628 that allow objective assessment of both first ray mobility and first ray position, improving efficiency of related clinical procedures. In contrast, conventional devices are generally only operable to assess first ray mobility but not first ray position. Additionally, conventional devices generally require at least some form of manual force during examination, potentially adversely affecting reliability and consistency of the result. One advantage of using an actuation member and vertical measurement mechanism is the ability to provide assessments of first ray mobility and position of a patient's foot that are more reliable, consistent, and objective compared with conventional devices.

A further advantage of the exemplary device embodiments is the inclusion of an arch height measurement mechanism and a torque member, *e.g.,* arch height measurement mechanism 616 and torque member 620, that allow contemporaneous assessment of arch height index, arch height flexibility, arch stiffness, and first MTP joint flexibility. In contrast, conventional devices do not allow for assessment of a range of foot structure and function, including first ray mobility, first ray position, arch height index, arch height flexibility, arch stiffness, and first MTP joint flexibility, let alone contemporaneously using a single device.

Furthermore, the exemplary device embodiments advantageously include a horizontal measurement mechanism, a foot length measurement mechanism, and an actuation member having a position sensor, *e.g.,* horizontal measurement mechanism 110, 610 foot length measurement mechanism 618, and actuation member 202, 650 that allow for measurement of a foot length of a patient's foot including truncated foot length and total foot length. Accordingly, the exemplary device embodiments allow for normalization of the foot structure and function assessments instead of raw measurements, *e.g.,* by determining a first ray mobility index, a first ray position index, and an arch height index, yielding assessments that are effective for a wide variety of foot sizes.

It will be appreciated by those skilled in the art that changes could be made to the various aspects described above without departing from the broad inventive concept thereof. It is to be understood, therefore, that the subject application is not limited to the particular aspects disclosed, but it is intended to cover modifications within the scope of the subject application as defined by the appended claims.

**Claims**

1.    A foot structure and function assessment device (100) comprising :

   a base (102) having a top surface (104) for supporting a patient's foot (126),
   a clamp (106) attached to the base configured to immobilize a second metatarsal of the patient's foot;
   a vertical measurement mechanism (128) carried by the base configured to measure displacement of a first ray of the patient's foot;
   an actuation member (202) configured to vertically displace the first ray, the actuation member including a first movable pointer (130) adjacent the vertical measurement mechanism configured to measure a first ray position of the patient's foot, and a second movable pointer (132) adjacent the vertical measurement mechanism configured to measure a first ray mobility of the patient's foot; and
   a controller (206) operatively in communication with the actuation member and configured to:

      apply a first vertical load to the first ray using the actuation member for displacing the first ray while the second metatarsal is immobilized, and determine the first ray position of the patient's foot under the first vertical load, and
      apply a second vertical load to the first ray using the actuation member for displacing the first ray while the second metatarsal is immobilized, and determine the first ray mobility of the patient's foot under the second vertical load.

2.    The foot structure and function assessment device of claim 1, wherein the first vertical load is about 3 N to about 10 N, and wherein the second vertical load is about 45 N to about 55 N.

3.    The foot structure and function assessment device of claim 1 or 2, further comprising:

   a heel cup (108) movably attached to the base for contacting a patient's rearfoot; and
   a horizontal measurement mechanism (110) carried by the base for measuring a foot length of the patient's foot.

4.    The foot structure and function assessment device of any one of claims 1 to 3, further comprising an actuator (114) rigidly coupled to the actuation member.

5.    The foot structure and function assessment device of any one of claims 1 to 4, wherein the clamp is laterally spaced from the actuation member, or wherein the clamp is spaced from the actuation member such that the clamp spatially corresponds to and is operable to immobilize a second metatarsal of the patient's foot as the actuation member displaces the first ray.

6.    The foot structure and function assessment device of any one of claims 1 to 5, wherein the actuation member is configured to contact a plantar surface of the first ray, or wherein the vertical measurement mechanism includes a vertical graticule, a position sensor, or a linear variable differential transformer.

7. The foot structure and function assessment device of any one of claims 1 to 6, further comprising an arch height measurement mechanism (616) laterally spaced from the actuation member and movable about a length of the patient's foot for measuring an arch height of the patient's foot.

8. The foot structure and function assessment device of any one of claims 1 to 7, further comprising a torque member (620) laterally spaced from the actuation member for applying a moment to the patient's foot about an axis of rotation of a first metatarsophalangeal joint.

9. The foot structure and function assessment device of any one of claims 1 to 8, wherein the controller is further configured to measure the first ray position of the patient's foot under the applied first vertical load, and to measure the first ray mobility of the patient's foot under the applied second vertical load.

10. A method (700) for assessing foot structure and function by using a foot structure and function assessment device (100) according to any one of preceding claims, comprising:

immobilizing (710), using the clamp (106), a second metatarsal of a patient's foot (126);
applying (720), by the controller (206) a first vertical load to a first ray of a patient's foot (126) using the actuation member (202) while the second metatarsal is immobilized, for displacing the first ray;
measuring (730), using the vertical measurement mechanism (128) and a first movable pointer (130) adjacent the vertical measurement mechanism (128), a first ray position of the patient's foot (126) under the applied first vertical load;
applying (740), by the controller (206) using the actuation member (202), a second vertical load to the first ray while the second metatarsal is immobilized, for displacing the first ray; and
measuring (750), using the vertical measurement mechanism (128) and the second movable pointer (132) adjacent the vertical measurement mechanism (128), a first ray mobility of the patient's foot (126) under the applied second vertical load.

11. The method of claim 10, wherein the applied first vertical load is about 5 N and wherein the applied second vertical load is about 50 N.

12. The method of any claim 10 or 11, further comprising:

measuring, using a foot length measurement mechanism (618) or a position sensor (615) coupled to the actuation member, a foot length of the patient's foot;
measuring, using an arch height measurement mechanism (616) laterally spaced from the actuation member, an arch height of the patient's foot; and
determining an arch height index of the patient's foot based on normalizing the arch height using the foot length.

13. The method of any one of claims 10 to 12, further comprising:

determining, using an arch height measurement mechanism (616) laterally spaced from the actuation member, an arch height of the patient's foot while the patient is seated;
determining, using the arch height measurement mechanism, an arch height of the patient's foot while the patient is standing;
measuring, using a scale (601) laterally spaced from the actuation member, a body weight of the patient; and
determining an arch height flexibility of the patient's foot based on the seated arch height, the standing arch height, and the body weight.

14. The method of any one of claims 10 to 13, further comprising:

determining, using an arch height measurement mechanism (616) laterally spaced from the actuation member, an arch height index of the patient's foot while the patient is seated;
determining, using the arch height measurement mechanism, an arch height index of the patient's foot while the patient is standing;
measuring, using a scale (601) laterally spaced from the actuation member, a body weight of the patient; and
determining an arch stiffness of the patient's foot based on the body weight, the seated arch height index, and the standing arch height index.

15. The method of any one of claims 10 to 14, further comprising:

applying, using a torque member (620) laterally spaced from the actuation member, a joint moment to a first metatarsophalangeal joint of the patient's foot; and

determining a first metatarsophalangeal joint flexibility of the patient's foot under the applied joint moment, wherein the determining the first metatarsophalangeal joint flexibility comprises determining the first metatarsophalangeal joint flexibility based on determining a slope (1210, 1220) of a first metatarsophalangeal joint flexibility curve (1200), or wherein the determining the first metatarsophalangeal joint flexibility comprises determining an early first metatarsophalangeal joint flexibility or a late first metatarsophalangeal joint flexibility.

**Patentansprüche**

1. Fußstruktur- und Funktionsbeurteilungsvorrichtung (100), umfassend eine Basis (102), die eine obere Fläche (104) zum Stützen des Fußes (126) eines Patienten aufweist,

eine Klemme (106), die an der Basis befestigt ist und konfiguriert ist, um einen zweiten Mittelfußknochen des Fußes des Patienten zu immobilisieren;

einen vertikalen Messmechanismus (128), der von der Basis getragen wird und konfiguriert ist, um eine Verschiebung eines ersten Strahls des Fußes des Patienten zu messen;

ein Betätigungselement (202), das konfiguriert ist, um den ersten Strahl vertikal zu verschieben, wobei das Betätigungselement einen ersten beweglichen Zeiger (130) neben dem vertikalen Messmechanismus, der dazu konfiguriert ist, eine erste Strahlposition des Fußes des Patienten zu messen, und einen zweiten beweglichen Zeiger (132) neben dem vertikalen Messmechanismus, der dazu konfiguriert ist, eine erste Strahlbeweglichkeit des Fußes des Patienten zu messen, beinhaltet; und

eine Steuerung (206), die funktionsfähig in Kommunikation mit dem Betätigungselement ist und zu Folgendem konfiguriert ist:

Aufbringen einer ersten vertikalen Belastung auf den ersten Strahl unter Verwendung des Betätigungselements zum Verschieben des ersten Strahls, während der zweite Mittelfußknochen immobilisiert ist, und Bestimmen der ersten Strahlposition des Fußes des Patienten unter der ersten vertikalen Belastung, und Aufbringen einer zweiten vertikalen Last auf den ersten Strahl unter Verwendung des Betätigungselements zum Verschieben des ersten Strahls, während der zweite Mittelfußknochen immobilisiert ist, und Bestimmen der Beweglichkeit des Fußes des ersten Strahls des Patienten unter der zweiten vertikalen Last.

2. Fußstruktur- und Funktionsbeurteilungsvorrichtung nach Anspruch 1, wobei die erste vertikale Last etwa 3 N bis etwa 10 N ist und wobei die zweite vertikale Last etwa 45 N bis etwa 55 N ist.

3. Fußstruktur- und Funktionsbeurteilungsvorrichtung nach Anspruch 1 oder 2, ferner umfassend:

eine Fersenschale (108), die bewegbar an der Basis angebracht ist, um die Fußhinterseite eines Patienten zu berühren; und

einen horizontalen Messmechanismus (110), der von der Basis getragen wird, um eine Fußlänge des Fußes des Patienten zu messen.

4. Fußstruktur- und Funktionsbeurteilungsvorrichtung nach einem der Ansprüche 1 bis 3, ferner umfassend einen Aktuator (114), der starr mit dem Betätigungselement gekoppelt ist.

5. Fußstruktur- und Funktionsbeurteilungsvorrichtung nach einem der Ansprüche 1 bis 4, wobei die Klemme seitlich von dem Betätigungselement beabstandet ist, oder wobei die Klemme von dem Betätigungselement beabstandet ist, sodass die Klemme räumlich einem zweiten Mittelfußknochen des Fußes des Patienten entspricht und betreibbar ist, um diesen zu immobilisieren, wenn das Betätigungselement den ersten Strahl verschiebt.

6. Fußstruktur- und Funktionsbeurteilungsvorrichtung nach einem der Ansprüche 1 bis 5, wobei das Betätigungselement dazu konfiguriert ist, eine Plantarfläche des ersten Strahls zu berühren, oder wobei der vertikale Messmechanismus ein vertikales Fadenkreuz, einen Positionssensor oder einen linearen variablen Differentialtransformator beinhaltet.

7. Fußstruktur- und Funktionsbeurteilungsvorrichtung nach einem der Ansprüche 1 bis 6, ferner umfassend einen Gewölbehöhenmessmechanismus (616), der seitlich von dem Betätigungselement beabstandet ist und um eine Länge des Fußes des Patienten beweglich ist, um eine Gewölbehöhe des Fußes des Patienten zu messen.

8. Fußstruktur- und Funktionsbeurteilungsvorrichtung nach einem der Ansprüche 1 bis 7, ferner umfassend ein Drehmomentelement (620), das seitlich von dem Betätigungselement beabstandet ist, um ein Moment auf den Fuß des Patienten um eine Drehachse eines ersten Großzehengrundgelenks anzuwenden.

9. Fußstruktur- und Funktionsbeurteilungsvorrichtung nach einem der Ansprüche 1 bis 8, wobei die Steuerung ferner konfiguriert ist, um die erste Strahlenposition des Fußes des Patienten unter der aufgebrachten ersten vertikalen Belastung zu messen und die erste Strahlenbeweglichkeit des Fußes des Patienten unter der aufgebrachten zweiten vertikalen Belastung zu messen.

10. Verfahren (700) zum Beurteilen von Fußstruktur und -funktion durch Verwenden einer Fußstruktur- und Funktionsbeurteilungsvorrichtung (100) nach einem der vorherigen Ansprüche, umfassend:

Immobilisieren (710), unter Verwendung der Klemme (106), eines zweiten Mittelfußknochens eines Patientenfußes (126);
Aufbringen (720), durch die Steuerung (206), einer ersten vertikalen Last auf einen ersten Strahl eines Patientenfußes (126) unter Verwendung des Betätigungselements (202), während der zweite Mittelfußknochen immobilisiert ist, um den ersten Strahl zu verschieben;
Messen (730), unter Verwendung des vertikalen Messmechanismus (128) und eines ersten beweglichen Zeigers (130) neben dem vertikalen Messmechanismus (128), einer ersten Strahlenposition des Fußes (126) des Patienten unter der aufgebrachten ersten vertikalen Last;
Aufbringen (740), durch die Steuerung (206) unter Verwendung des Betätigungselements (202), einer zweiten vertikalen Last auf den ersten Strahl, während der zweite Mittelfußknochen immobilisiert ist, um den ersten Strahl zu verschieben; und
Messen (750), unter Verwendung des vertikalen Messmechanismus (128) und des zweiten beweglichen Zeigers (132) neben dem vertikalen Messmechanismus (128), einer ersten Strahlbeweglichkeit des Fußes (126) des Patienten unter der aufgebrachten zweiten vertikalen Last.

11. Verfahren nach Anspruch 10, wobei die aufgebrachte erste vertikale Last etwa 5 N ist und wobei die aufgebrachte zweite vertikale Last etwa 50 N ist.

12. Verfahren nach Anspruch 10 oder 11, ferner umfassend:

Messen, unter Verwendung eines Fußlängenmessmechanismus (618) oder eines Positionssensors (615), der an das Betätigungselement gekoppelt ist, einer Fußlänge des Fußes des Patienten;
Messen, unter Verwendung eines Gewölbehöhenmessmechanismus (616), der seitlich von dem Betätigungselement beabstandet ist, einer Gewölbehöhe des Fußes des Patienten; und
Bestimmen eines Gewölbehöhenindex des Fußes des Patienten basierend auf der Normalisierung der Gewölbehöhe unter Verwendung der Fußlänge.

13. Verfahren nach einem der Ansprüche 10 bis 12, ferner umfassend:

Bestimmen, unter Verwendung eines Gewölbehöhenmessmechanismus (616), der seitlich von dem Betätigungselement beabstandet ist, einer Gewölbehöhe des Fußes des Patienten, während der Patient sitzt;
Bestimmen, unter Verwendung des Gewölbehöhenmessmechanismus, einer Gewölbehöhe des Fußes des Patienten, während der Patient steht;
Messen eines Körpergewichts des Patienten unter Verwendung einer Skala (601), die seitlich von dem Betätigungselement beabstandet ist; und
Bestimmen einer Gewölbehöhenflexibilität des Fußes des Patienten basierend auf der sitzenden Gewölbehöhe, der stehenden Gewölbehöhe und des Körpergewichts.

14. Verfahren nach einem der Ansprüche 10 bis 13, ferner umfassend:

Bestimmen, unter Verwendung eines Gewölbehöhenmessmechanismus (616), der seitlich von dem Betätigungselement beabstandet ist, eines Gewölbehöhenindex des Fußes des Patienten, während der Patient sitzt;

Bestimmen, unter Verwendung des Gewölbehöhenmessmechanismus, eines Gewölbehöhenindex des Fußes des Patienten, während der Patient steht;

Messen eines Körpergewichts des Patienten unter Verwendung einer Skala (601), die seitlich von dem Betätigungselement beabstandet ist; und

Bestimmen einer Fußgewölbesteifigkeit des Fußes des Patienten basierend auf dem Körpergewicht, dem sitzenden Gewölbehöhenindex und dem stehenden Gewölbehöhenindex.

**15.** Verfahren nach einem der Ansprüche 10 bis 14, ferner umfassend:

Aufbringen, unter Verwendung eines Drehmomentelements (620), das seitlich von dem Betätigungselement beabstandet ist, eines Gelenkmoments auf ein erstes Großzehengrundgelenk des Fußes des Patienten; und

Bestimmen einer ersten Großzehengrundgelenkflexibilität des Fußes des Patienten unter dem aufgebrachten Gelenkmoment, wobei das Bestimmen der ersten Großzehengrundgelenkflexibilität ein Bestimmen der ersten Großzehengrundgelenkflexibilität basierend auf einem Bestimmen einer Neigung (1210, 1220) einer ersten Großzehengrundgelenk-Flexibilitätskurve (1200) umfasst, oder wobei das Bestimmen der ersten Großzehengrundgelenkflexibilität ein Bestimmen einer frühen ersten Großzehengrundgelenkflexibilität oder einer späten ersten Großzehengrundgelenkflexibilität umfasst.

**Revendications**

**1.** Dispositif d'évaluation de structure et de fonction de pied (100) comprenant une base (102) présentant une surface supérieure (104) pour supporter le pied (126) d'un patient, une pince (106) fixée à la base, configurée pour immobiliser un deuxième métatarse du pied du patient ;

un mécanisme de mesure vertical (128) porté par la base, configuré pour mesurer le déplacement d'un premier rayon du pied du patient ;

un élément d'actionnement (202) configuré pour déplacer verticalement le premier rayon, l'élément d'actionnement incluant un premier pointeur mobile (130) adjacent au mécanisme de mesure vertical configuré pour mesurer une position de premier rayon du pied du patient, et un second pointeur mobile (132) adjacent au mécanisme de mesure vertical configuré pour mesurer une mobilité de premier rayon du pied du patient ; et

un dispositif de commande (206) en communication fonctionnelle avec l'élément d'actionnement et configuré pour :

appliquer une première charge verticale au premier rayon à l'aide de l'élément d'actionnement pour déplacer le premier rayon en même temps que le deuxième métatarse est immobilisé, et déterminer la position de premier rayon du pied du patient sous la première charge verticale, et

appliquer une seconde charge verticale au premier rayon à l'aide de l'élément d'actionnement pour déplacer le premier rayon en même temps que le deuxième métatarse est immobilisé, et déterminer la mobilité de premier rayon du pied du patient sous la seconde charge verticale.

**2.** Dispositif d'évaluation de structure et de fonction de pied selon la revendication 1, dans lequel la première charge verticale est d'environ 3 N à environ 10 N, et dans lequel la seconde charge verticale est d'environ 45 N à environ 55 N.

**3.** Dispositif d'évaluation de structure et de fonction de pied selon la revendication 1 ou 2, comprenant en outre :

une coque talonnière (108) fixée de manière mobile à la base pour entrer en contact avec l'arrière-pied d'un patient ; et

un mécanisme de mesure horizontal (110) porté par la base pour mesurer une longueur de pied du pied du patient.

**4.** Dispositif d'évaluation de structure et de fonction de pied selon l'une quelconque des revendications 1 à 3, comprenant en outre un actionneur (114) couplé de manière rigide à l'élément d'actionnement.

**5.** Dispositif d'évaluation de structure et de fonction de pied selon l'une quelconque des revendications 1 à 4, dans lequel la pince est espacée latéralement de l'élément d'actionnement, ou dans lequel la pince est espacée de l'élément d'actionnement de sorte que la pince corresponde spatialement à et puisse fonctionner pour immobiliser un deuxième métatarse du pied du patient lorsque l'élément d'actionnement déplace le premier rayon.

6. Dispositif d'évaluation de structure et de fonction de pied selon l'une quelconque des revendications 1 à 5, dans lequel l'élément d'actionnement est configuré pour entrer en contact avec une surface plantaire du premier rayon, ou dans lequel le mécanisme de mesure vertical inclut un repère vertical, un capteur de position ou un transformateur différentiel variable linéaire.

7. Dispositif d'évaluation de structure et de fonction de pied selon l'une quelconque des revendications 1 à 6, comprenant en outre un mécanisme de mesure de hauteur de voûte plantaire (616) espacé latéralement de l'élément d'actionnement et mobile sur une longueur du pied du patient pour mesurer une hauteur de voûte plantaire du pied du patient.

8. Dispositif d'évaluation de structure et de fonction de pied selon l'une quelconque des revendications 1 à 7, comprenant en outre un élément de couple (620) espacé latéralement de l'élément d'actionnement pour appliquer un moment au pied du patient autour d'un axe de rotation d'une première articulation métatarso-phalangienne.

9. Dispositif d'évaluation de structure et de fonction de pied selon l'une quelconque des revendications 1 à 8, dans lequel le dispositif de commande est en outre configuré pour mesurer la position de premier rayon du pied du patient sous la première charge verticale appliquée, et pour mesurer la mobilité de premier rayon du pied du patient sous la seconde charge verticale appliquée.

10. Procédé (700) d'évaluation de structure et de fonction de pied à l'aide d'un dispositif d'évaluation de structure et de fonction de pied (100) selon l'une quelconque des revendications précédentes, comprenant :

   l'immobilisation (710), à l'aide de la pince (106), d'un deuxième métatarse du pied (126) d'un patient ;
   l'application (720), par le dispositif de commande (206), d'une première charge verticale à un premier rayon du pied (126) du patient à l'aide de l'élément d'actionnement (202) en même temps que le deuxième métatarse est immobilisé, pour déplacer le premier rayon ;
   la mesure (730), à l'aide du mécanisme de mesure vertical (128) et d'un premier pointeur mobile (130) adjacent au mécanisme de mesure vertical (128), d'une position de premier rayon du pied (126) du patient sous la première charge verticale appliquée ;
   l'application (740), par le dispositif de commande (206) à l'aide de l'élément d'actionnement (202), d'une seconde charge verticale au premier rayon en même temps que le deuxième métatarse est immobilisé, pour déplacer le premier rayon ; et
   la mesure (750), à l'aide du mécanisme de mesure vertical (128) et du second pointeur mobile (132) adjacent au mécanisme de mesure vertical (128), d'une mobilité de premier rayon du pied (126) du patient sous la seconde charge verticale appliquée.

11. Procédé selon la revendication 10, dans lequel la première charge verticale appliquée est d'environ 5 N et dans lequel la seconde charge verticale appliquée est d'environ 50 N.

12. Procédé selon la revendication 10 ou 11, comprenant en outre :

   la mesure, à l'aide d'un mécanisme de mesure de longueur de pied (618) ou d'un capteur de position (615) couplé à l'élément d'actionnement, d'une longueur de pied du pied du patient ;
   la mesure, à l'aide d'un mécanisme de mesure de hauteur de voûte plantaire (616) espacé latéralement de l'élément d'actionnement, d'une hauteur de voûte plantaire du pied du patient ; et
   la détermination d'un indice de hauteur de voûte plantaire du pied du patient en fonction de la normalisation de la hauteur de voûte plantaire en utilisant la longueur de pied.

13. Procédé selon l'une quelconque des revendications 10 et 12, comprenant en outre :

   la détermination, à l'aide d'un mécanisme de mesure de hauteur de voûte plantaire (616) espacé latéralement de l'élément d'actionnement, d'une hauteur de voûte plantaire du pied du patient lorsque le patient est assis ;
   la détermination, à l'aide du mécanisme de mesure de hauteur de voûte plantaire, d'une hauteur de voûte plantaire du pied du patient lorsque le patient est debout ;
   la mesure, à l'aide d'une balance (601) espacée latéralement de l'élément d'actionnement, d'un poids corporel du patient ; et
la détermination d'une souplesse de hauteur de voûte plantaire du pied du patient en fonction de la hauteur de voûte plantaire assis, de la hauteur de voûte plantaire debout et du poids corporel.

14. Procédé selon l'une quelconque des revendications 10 et 13, comprenant en outre :

la détermination, à l'aide d'un mécanisme de mesure de hauteur de voûte plantaire (616) espacé latéralement de l'élément d'actionnement, d'un indice de hauteur de voûte plantaire du pied du patient lorsque le patient est assis ;
la détermination, à l'aide du mécanisme de mesure de hauteur de voûte plantaire, d'un indice de hauteur de voûte plantaire du pied du patient lorsque le patient est debout ;
la mesure, à l'aide d'une balance (601) espacée latéralement de l'élément d'actionnement, d'un poids corporel du patient ; et
la détermination d'une raideur de voûte plantaire du pied du patient sur la base du poids corporel, de l'indice de hauteur de voûte plantaire assis et de l'indice de hauteur de voûte plantaire debout.

15. Procédé selon l'une quelconque des revendications 10 et 14, comprenant en outre :

l'application, à l'aide d'un élément de couple (620) espacé latéralement de l'élément d'actionnement, d'un moment articulaire à une première articulation métatarso-phalangienne du pied du patient ; et
la détermination d'une souplesse de première articulation métatarso-phalangienne du pied du patient sous le moment articulaire appliqué,
dans lequel la détermination de la souplesse de première articulation métatarso-phalangienne comprend la détermination de la souplesse de première articulation métatarso-phalangienne sur la base de la détermination d'une pente (1210, 1220) d'une courbe (1200) de souplesse de première articulation métatarso-phalangienne,
ou dans lequel la détermination de la souplesse de première articulation métatarso-phalangienne comprend la détermination d'une souplesse initiale de première articulation métatarso-phalangienne ou d'une souplesse finale de première articulation métatarso-phalangienne.

FIG. 1

FIG. 2

FIG. 3

FIG. 4

FIG. 5

FIG. 6

700

| 710 | IMMOBILIZE SECOND METATARSAL OF PATIENT'S FOOT USING CLAMP |
|---|---|
| 720 | DISPLACE FIRST RAY BY APPLYING FIRST VERTICAL LOAD TO FIRST RAY OF PATIENT'S FOOT USING ACTUATION MEMBER |
| 730 | MEASURE FIRST RAY POSITION UNDER APPLIED FIRST VERTICAL LOAD |
| 740 | DISPLACE FIRST RAY BY APPLYING SECOND VERTICAL LOAD TO FIRST RAY USING ACTUATION MEMBER |
| 750 | MEASURE FIRST RAY MOBILITY UNDER APPLIED SECOND VERTICAL LOAD |

FIG. 7

*800*

*810* — | MEASURE FOOT LENGTH USING FOOT LENGTH MEASUREMENT MECHANISM AND/OR ACTUATION MEMBER |

*820* — | MEASURE ARCH HEIGHT USING ARCH HEIGHT MEASUREMENT MECHANISM |

*830* — | DETERMINE ARCH HEIGHT INDEX BASED ON NORMALIZING ARCH HEIGHT USING FOOT LENGTH |

FIG. 8

900

910 — DETERMINE ARCH HEIGHT USING ARCH HEIGHT MEASUREMENT MECHANISM WHILE PATIENT IS SEATED

920 — DETERMINE ARCH HEIGHT USING ARCH HEIGHT MEASUREMENT MECHANISM WHILE PATIENT IS STANDING

930 — MEASURE BODY WEIGHT USING SCALE

940 — DETERMINE ARCH HEIGHT FLEXIBILITY BASED ON SEATED ARCH HEIGHT, STANDING ARCH HEIGHT, AND BODY WEIGHT

FIG. 9

FIG. 10

1100

1110 — IMMOBILIZE SECOND METATARSAL OF
PATIENT'S FOOT USING CLAMP

1120 — APPLY JOINT MOMENT TO FIRST MTP JOINT OF
PATIENT'S FOOT USING TORQUE MEMBER

1130 — DETERMINE FIRST MTP JOINT FLEXIBILITY
UNDER APPLIED MOMENT

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017171660 A1

**Non-patent literature cited in the description**

- **GLASOE WARD MYLO et al.** The Reliability and Validity of a First Ray Measurement Device. *FOOT AND ANKLE INTERNATIONAL*, 01 March 2000, vol. 21 (3), ISBN
ISSN: 1071-1007, DOI: 10.1177/107110070002100310, 240-246